# EUROPEAN PATENT SPECIFICATION

(11) **EP 1 578 272 B1**
(45) Date of publication and mention of the grant of the patent: **30.12.2015**
(21) Application number: 03789086.0
(22) Date of filing: 26.11.2003
(51) Int. Cl.: A61B 5/155, G01N 33/487

(54) **BODY FLUID TESTING DEVICE**
TESTGERÄT FÜR KÖRPERFLÜSSIGKEIT
DISPOSITIF DE TEST DE LIQUIDE BIOLOGIQUE

(30) Priority: 26.11.2002 EP 02026242
(43) Date of publication of application: 28.09.2005
(73) Proprietor: Roche Diagnostics GmbH, 68305 Mannheim (DE); F. Hoffmann-La Roche AG, 4070 Basel (CH)
(72) Inventor: ZIMMER, Volker, 54497 Morbach (DE); HAAR, Hans-Peter, 69168 Wiesloch (DE); MANSER, Maria, 68723 Schwetzingen (DE); HOENES, Joachim, 64673 Zwingenberg (DE); MILTNER, Karl, 67227 Frankenthal (DE); LIST, Hans, 64754 Hesseneck-Kailbach (DE); PACHL, Rudolf, 67158 Ellerstadt (DE)
(86) International application number: PCT/EP2003/013298
(87) International publication number: WO 2004/047642

(56) References cited:
- WO-A-01/08551
- DE-A- 10 105 549
- DE-A- 19 819 407
- DE-A- 19 849 539
- DE-A- 19 857 426
- US-A- 5 228 972
- US-A- 5 686 829

## Description

### BACKGROUND OF THE INVENTION

The present invention relates to body fluid testing devices and more specifically, but not exclusively, concerns a body fluid testing device that incorporates a test media cassette which contains test media used to test body fluid.

### General Fluid Testing

The acquisition and testing of body fluids is useful for many purposes, and continues to grow in importance for use in medical diagnosis and treatment, and in other diverse applications. In the medical field, it is desirable for lay operators to perform tests routinely, quickly and reproducibly outside of a laboratory setting, with rapid results and a readout of the resulting test information. Testing can be performed on various body fluids, and for certain applications is particularly related to the testing of blood and/or interstitial fluid. Such fluids can be tested for a variety of characteristics of the fluid, or analytes contained in the fluid, in order to identify a medical condition, determine therapeutic responses, assess the progress of treatment, and the like.

### General Test Steps

The testing of body fluids basically involves the steps of obtaining the fluid sample, transferring the sample to a test device, conducting a test on the fluid sample, and displaying the results. These steps are generally performed by a plurality of separate instruments or devices.

### Acquiring - Vascular

One method of acquiring the fluid sample involves inserting a hollow needle or syringe into a vein or artery in order to withdraw a blood sample. However, such direct vascular blood sampling can have several limitations, including pain, infection, and hematoma and other bleeding complications. In addition, direct vascular blood sampling is not suitable for repeating on a routine basis, can be extremely difficult and is not advised for patients to perform on themselves.

### Acquiring - Incising

The other common technique for collecting a body fluid sample is to form an incision in the skin to bring the fluid to the skin surface. A lancet, knife or other cutting instrument is used to form the incision in the skin. The resulting blood or interstitial fluid specimen is then collected in a small tube or other container, or is placed directly in contact with a test strip. The fingertip is frequently used as the fluid source because it is highly vascularized and therefore produces a good quantity of blood. However, the fingertip also has a large concentration of nerve endings, and lancing the fingertip can therefore be painful. Alternate sampling sites, such as the palm of the hand, forearm, earlobe and the like, may be useful for sampling, and are less painful. However, they also produce lesser amounts of blood. These alternate sites therefore are generally appropriate for use only for test systems requiring relatively small amounts of fluid, or if steps are taken to facilitate the expression of the body fluid from the incision site.

Various methods and systems for incising the skin are known in the art. Exemplary lancing devices are shown, for example, in United States Patent Nos. Re 35,803, issued to Lange, et al. on May 19,1998.; 4,924,879, issued to O'Brien on May 15, 1990; 5,879,311, issued to Duchon et al. on February 16, 1999; 5,857,983, issued to Douglas on January 12, 1999; 6,183,489, issued to Douglas et al. on February 6, 2001; 6,332,871, issued to Douglas et al. on December 25, 2001; and 5,964,718, issued to Duchon et al. on October 12, 1999. A representative commercial lancing device is the Accu-Chek Softclix lancet.

### Expressing

Patients are frequently advised to urge fluid to the incision site, such as by applying pressure to the area surrounding the incision to milk or pump the fluid from the incision. Mechanical devices are also known to facilitate the expression of body fluid from an incision. Such devices are shown, for example, in United States Patent Nos. 5,879,311, issued to Duchon et al. on February 16, 1999; 5,857,983, issued to Douglas on January 12, 1999; 6,183,489, issued to Douglas et al. on February 6, 2001; 5,951,492, issued to Douglas et al. on September 14, 1999; 5,951,493, issued to Douglas et al. on September 14, 1999; 5,964,718, issued to Duchon et al. on October 12, 1999; and 6,086,545, issued to Roe et al. on July 11, 2000. A representative commercial product that promotes the expression of body fluid from an incision is the Amira AtLast blood glucose system.

### Sampling

The acquisition of the produced body fluid, hereafter referred to as the "sampling" of the fluid, can take various forms. Once the fluid specimen comes to the skin surface at the incision, a sampling device is placed into contact with the fluid. Such devices may include, for example, systems in which a tube or test strip is either located adjacent the incision site prior to forming the incision, or is moved to the incision site shortly after the incision has been formed. A sampling tube may acquire the fluid by suction or by capillary action. Such sampling systems may include, for example, the systems shown in US Patent Nos. 6,048,352, issued to Douglas et al. on April 11, 2000; 6,099,484, issued to Douglas et al. on August 8, 2000; and 6,332,871, issued to Douglas et al. on December 25, 2001. Examples of commercial sampling devices include the Roche Compact, Amira AtLast, Glucometer Elite and Therasense FreeStyle test strips.

### Testing General

The body fluid sample may be analyzed for a variety of properties or components, as is well known in the art. For example, such analysis may be directed to hematocrit, blood glucose, coagulation, lead, iron, etc. Testing systems include such means as optical (e.g., reflectance, absorption, fluorescence, Raman, etc.), electrochemical, and magnetic means for analyzing the sampled fluid. Examples of such test systems include those in US Patent Nos. 5,824,491, issued to Priest et al. on October 20, 1998; 5,962,215, issued to Douglas et al. on October 5, 1999; and 5,776,719, issued to Douglas et al. on July 7,1998.

Typically, a test system takes advantage of a reaction between the body fluid to be tested and a reagent present in the test system. For example, an optical test strip will generally rely upon a color change, i.e., a change in the wavelength absorbed or reflected by dye formed by the reagent system used. See, e.g., US Patent Nos. 3,802,842; 4,061,468; and 4,490,465.

### Blood Glucose

A common medical test is the measurement of blood glucose level. The glucose level can be determined directly by analysis of the blood, or indirectly by analysis of other fluids such as interstitial fluid. Diabetics are generally instructed to measure their blood glucose level several times a day, depending on the nature and severity of their diabetes. Based upon the observed pattern in the measured glucose levels, the patient and physician determine the appropriate level of insulin to be administered, also taking into account such issues as diet, exercise and other factors.

In testing for the presence of an analyte such as glucose in a body fluid, test systems are commonly used which take advantage of an oxidation/reduction reaction which occurs using an oxidase/peroxidase detection chemistry. The test reagent is exposed to a sample of the body fluid for a suitable period of time, and there is a color change if the analyze (glucose) is present. Typically, the intensity of this change is proportional to the concentration of analyte in the sample. The color of the reagent is then compared to a known standard which enables one to determine the amount of analyte present in the sample. This determination can be made, for example, by a visual check or by an instrument, such as a reflectance spectrophotometer at a selected wavelength, or a blood glucose meter. Electrochemical and other systems are also well known for testing body fluids for properties on constituents.

### Testing Media

As mentioned above, diabetics typically have to monitor their blood glucose levels throughout the day so as to ensure that their blood glucose remains within an acceptable range. Some types sampling devices require the use of testing strips that contain media for absorbing and/or testing the body fluid, such as blood. After testing, the testing media contaminated with blood can be considered a biohazard and needs to be readily disposed in order to avoid other individuals from being exposed to the contaminated test strip. This can be especially inconvenient when the person is away from home, such as at restaurant. Moreover, individual test elements can become easily mixed with other test strips having different expiration dates. The use of expired test elements may create false readings, which can result in improper treatment of the patient, such as improper insulin dosages for diabetics.

### Test Media Cassettes

Analytical systems with test media cassettes which allow multiple testing have been described in the prior art (see e.g. US 4,218,421 and US 5,077,010) for the environment of automated laboratory systems which use pipettes to apply sample fluid to the test media. These systems are therefore not suited to be used by lay people as e.g. diabetics for self testing. DE 198 19 407 describes a test element cassette for use in the home environment. Figure 1 describes a tape of electrochemical test elements having punched out regions that are presented for blood application when a test element of the tape is moved over a bended surface. Figure 2 of DE 198 19 407 shows a test cassette with a tape of test media for reflectometric analysis. Application of sample fluid, especially body fluid which is located at a body portion is cumbersome with the shown apparatus.

DE-198 49 539 and US-5,686,829 also show test media cassettes.

It was an aim of the present invention to facilitate user handling for body fluid testing.

### SUMMARY OF THE INVENTION

The present invention provides various devices and methods for testing body fluid. The present invention encompasses a body fluid testing device that contains a plurality of test media.

In accordance with one aspect of the present invention, there is provided a body fluid testing device for analyzing a body fluid. The testing device includes a test media cassette that includes a test media tape adapted to collect the body fluid. The cassette includes a supply portion that stores an uncontaminated section of the test media tape. A storage portion for storing a contaminated section of the test media tape is further employed.

The testing device is a handheld device that can be conveniently handled by an user. The test media tape may be a part of the testing device so that the whole device is discarded when the test media tape is used up or the test media tape may be arranged in a disposable cassette which is received in the testing device. An important aspect of a first embodiment of the present invention is that a portion of the test media tape onto which body fluid will be applied is exposed in a tip like shape. For this purpose the test media tape is guided over a convex tip portion which may belong to the testing device or to the test media cassette. Due to this tip portion body fluid can be applied to the exposed portion of the test media tape very conveniently.

A sensing region is positioned between the supply portion and the storage portion to sense at least one property or analyte of the body fluid collected on the test media tape at the exposure portion of the cassette.

The testing device further may comprise a pricking unit for pricking a body portion. The lancing opening of that pricking unit advantageously can be arranged in or close to the convex portion so that the tip portion can be used for convenient pricking as well. The pricking unit may be arranged below the test media tape and a lancing device can either penetrate the test media tape or can extend through a recess in the test media tape.

A further aspect of the present invention is a method and a device for visual user guidance for application of body fluid samples. According to this embodiment the testing device comprises an illumination unit which indicates by illumination of a portion of a test element where body fluid is to be applied. The illumination serves for a timely and / or spatially guidance of the user to apply body fluid. Further the illumination may serve to indicate the location where to position a body portion for pricking. An illuminated area on the test medium may further indicate the amount (or the droplet size) of body fluid which is required by the testing device. Another aspect of the present invention concerns a test cassette for collecting a body fluid sample. The cassette includes a test media tape, which has a section for receiving test media tape that is contaminated with past samples of the body fluid and a section for storing and providing uncontaminated test media tape. The cassette includes a housing that has a supply portion in which the uncontaminated section of the test media tape is enclosed. The housing further includes a storage portion in which the contaminated section of the test media tape is enclosed after contamination. The cassette further includes a convex tip portion over which the test media tape runs and at which the test media tape is exposed to the body fluid. A supply reel is disposed in the supply portion of the housing around which the uncontaminated section of the test media tape is wrapped. A storage reel is disposed in the storage portion of the housing around which the contaminated section of the test media tape is wrapped.

Further this invention concerns a method of using a testing device comprising the steps of
a) bringing a test element into a sample application position,
b) illuminating a portion of the test element onto which sample fluid application is desired,
c) application of sample fluid to the illuminated portion,
d) evaluation of a test medium of the test element to generate an analytical result
e) withdrawing the test element or the test medium from the sample application position.
In case of an embodiment employing a test media tape a number of test media are located on the same test element (e.g. a tape). The steps for bringing a test element into a sample application position and removing it from this position in this case are made by shifting the position of the test element. It further has to be understood that step e) can be made prior to step d) if evaluation is performed at a different position than sample application.
In case of a device receiving single use test elements an individual test element is brought into the sample application position and is taken out of the device after use.

A further step maybe included in the above method which concerns a pricking for generating a body opening.

Other forms, embodiments, objects, features, advantages, benefits and aspects of the present invention shall become apparent from the detailed drawings and description contained herein.

### Short description of the figures:

Figure 1: Testing devices having a tip portion at their distal end.
Figure 2: Blood application to a tip portion with a finger tip.
Figure 3: Tip portion embodiments in perspective view.
Figure 4: Cut trough a tip portion in plane with test media tape movement direction
Figure 5: Embodiments of supply and storage reel arrangements
Figure 6: Test media tape cassette with tip portion and testing device with inserted cassette
Figure 7: Test media tape cassette and testing device having a tip portion with inserted cassette
Figure 8: Embodiments employing illuminated sample application zones
Figure 9: Illumination concept applied to light guiding test element.
Figure 10: Illumination concept applied to capillary fill test element.

### DESCRIPTION OF SELECTED EMBODIMENTS

For the purposes of promoting an understanding of the principles of the invention, reference will now be made to the embodiments illustrated in the drawings and specific language will be used to describe the same. It will nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates. One embodiment of the invention is shown in great detail, although it will be apparent to those skilled in the art that some of the features which are not relevant to the invention may not be shown for the sake of clarity.

A first general concept of the present invention concerns a body fluid testing device that incorporates a test media tape. The test media tape holds test media that are used to collect body fluid samples which are analyzed with a sensor. Advantageously the test media tape is housed in a cassette so that after the test media of a cassette are used up a fresh test media cassette can be inserted into the testing device. The test media tape is indexed before or after each test so that successive tests can be performed without requiring disposal of individual test media. The test media can be indexed manually or automatically.
The test medium is a medium which contains a test chemistry that with analyte from a sample leads to detectable results. For further details of test chemistry and testing see section "Testing General". Preferably the test media are designed to soak up the test fluid sample. This prevents the testing device from becoming contaminated by body fluid sample. As will be described in more detail lateron it is preferred to employ a test media tape which comprises a transport tape on which test media are arranged with free spaces between successive test media. The preferred arrangement therefore has a structure with regions as follows: tape with test medium - tape without test medium - tape with test medium - and so on. The tape can be made e.g. from conventional plastic tape. The test media are attached to the tape, e.g. by glueing, welding or by use of an adhesive tape.

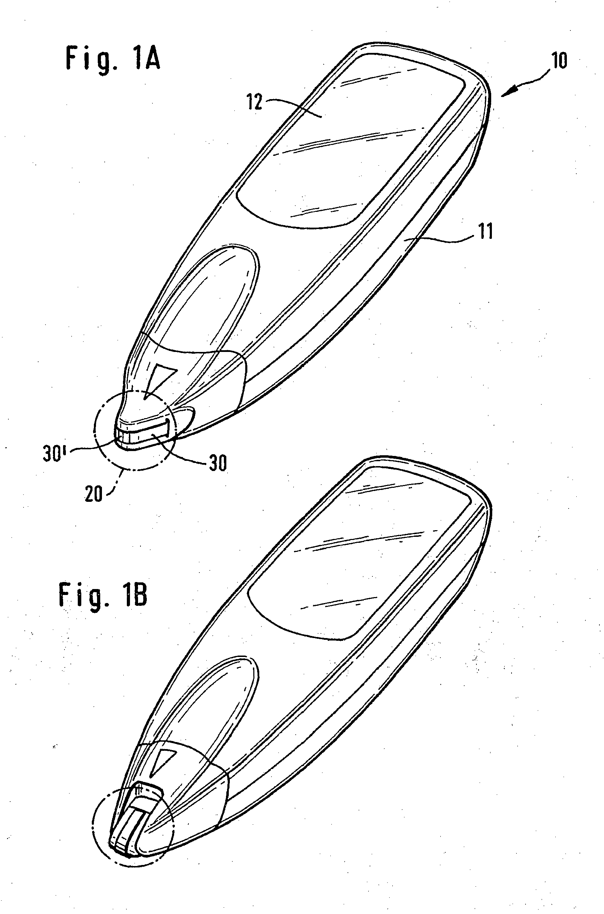

A body fluid testing device (10) according to the present invention is shown in figure 1A. The drawing of the device shows a housing (11) and a display (12) for displaying test results as well as instructions of use. At the front end of the device there can be seen a tip portion (20) over which the test media tape (30) runs. This tip portion is a first inventive idea of the present invention. A test medium at the front end of the testing device is exposed by the tip portion in a tip like manner which facilitates the application of body fluid. The tip portion for this reason at least partially projects out of the contour of the housing (11) of the testing device to be accessible for a body portion (e.g. finger or arm).

The testing device can be approached to a body surface (e.g. finger or arm) on which a body fluid sample is located with the tip portion. In order to make the tip portion easily accessible and visible as the location where sample has to be applied it is preferred to employ a tip like shape. Easy access and good visibility can be achieved by a tip portion that changes the direction of movement of the test media tape by an angle of more than 60 degree, preferably more than 90 degree.

Figure 1B shows a very similar testing device as depicted in figure 1A. While the test media tape (30) in the embodiment of figure 1A runs from the left to the right side of the device (or vice versa) in figure 1B the tape runs from the upper side to the lower side (or vice versa).

According to a second inventive concept of the present invention body fluid application can be further facilitated by illuminating a portion (30') of the test tape where the body fluid sample has to be applied. For this functionality a translucent test medium is advantageous. If the test medium, however, is opaque a translucent portion of the test media tape without test medium applied to it may be employed. However, most test media for analyte testing as e.g. reflectometric glucose test media are partially transparent and therefore produce an easily visible lighted area on the frontside of the test medium when illumination from the backside is made and the layer of test medium is not too thick. The artisan in this field knows how to make test media which transmit enough light that an illumination from the backside can easily be seen by a user. It is preferred to illuminate the backside of the test media tape with colored light (e.g. red or green) so that a better visible illumination results as by illumination with white light would result.

The second inventive concept of the present invention includes the user guidance by illuminating a portion of the test medium or a test element to which sample fluid has to be applied. As already described above illumination is made when the device is ready to receive sample fluid. Further the size of the illuminated area on the frontside of the test medium advantageously can be chosen to indicate the size of the test medium which needs to be filled with sample fluid to allow proper analysis. The user therefore can visually control whether he has applied enough sample fluid to the correct position on the test medium. The illumination for user guidance is made when the test element is positioned in the testing device for sample reception. For illumination the same optics as for optical evaluation can be used. However, if it is desired to indicate the size of sample fluid to be applied to the test medium it is preferred to employ a separate light source for this purpose or to employ means that change the size of illuminated area. For indication of application site and indication of desired sample volume in the latter case a first surface of the test medium is illuminated and a differently sized area (located within the first area) is illuminated for evaluating the test medium.

There is a certain connection between the area wetted by sample fluid and the recognition whether proper analysis can be done. Test media require a certain amount of sample volume to allow a reliable measurement. The amount of liquid which is necessary depends on a number of factors as the test chemistry, test architecture (layer structure with e.g. additional layers for removal of cells etc.), optics or electrodes and so on. For an actual testing device where these factors are fixed and the test medium is standardized the minimum amount of sample fluid needed for reliable measurement is a fixed number. On the other side the area on the test medium covered by sample fluid is related to the volume of sample fluid. In case of a non-absorbing test medium a drop is formed with an area that is dependent on surface tension. Based upon known surface conditions the area therefore can be related to the sample volume. In case of an absorbing test medium the absorption capacity per area determines the area which is wetted by a certain amount of fluid. The wetted area on the test medium therefore is closely related to the volume of fluid applied.
The testing device according to this second inventive idea illuminates an area of the test medium which becomes wetted when a sample volume equal or above the volume required for proper analysis is applied. The user therefore can visually control whether the fluid sample he has applied covers the whole illuminated area or not. This allows a twofold control
- if enough fluid has been applied
- if the body fluid sample has been applied to the correct location on the test medium.
Further it is advantageous to combine the user guidance by illumination with monitoring of fluid application to the test medium. Such monitoring is possible by optical or electrochemical detection. In an optical method light reflected from the test medium is detected and a change in intensity is monitored to detect sample fluid application. In an electrical process conductivity or capacity can be monitored to detect sample application. The before mentioned changes of measurement signals can be evaluated to give one or more of the following information:
- whether sample has been applied to the test medium,
- whether the sample has been applied to the correct position on the test medium
- whether the applied sample volume is sufficient.

Illumination at the tip portion further may serve to guide the user through the use process of the testing device. Blinking e.g. may indicate that the testing device is ready for sample fluid to be applied and a constant light or a deenergization of the light may show that sample has been successfully applied.

Illumination as described above can be implemented by employing a separate light source which is controlled by a control unit. However, in case of optical measurement as common in this field the light source for measurement can advantageously be employed for illumination of the test medium at the sampling position as well.

A testing device according to the above embodiment further has a control unit for controlling activation of the light source for illumination of the test medium. The control unit activates the light source when the instrument waits for fluid sample to be applied to the test medium. Illumination in this sense does not only mean constant illumination but also includes e.g. blinking. The illumination may remain until the user shuts down the testing device but it is preferred that the control unit deactivates illumination or changes the type of illumination when proper application of fluid to the test medium is detected (as described above). However, in case of optical evaluation of the test medium the illumination may be activated again for measuring an analyte concentration in the fluid sample.

The user guidance by illumination can be employed advantageously in embodiments where the test media portion for application of sample fluid is located at a tip. In this geometric setup the user can see the light when he is applying sample to the test medium. By sake of the tip geometry the user can visually monitor the application process, especially the approaching of the tip portion by a body portion on which body fluid is located. Due to illumination at the tip portion light is not shielded by the approaching body portion until shortly before contacting the illuminated area with sample fluid.

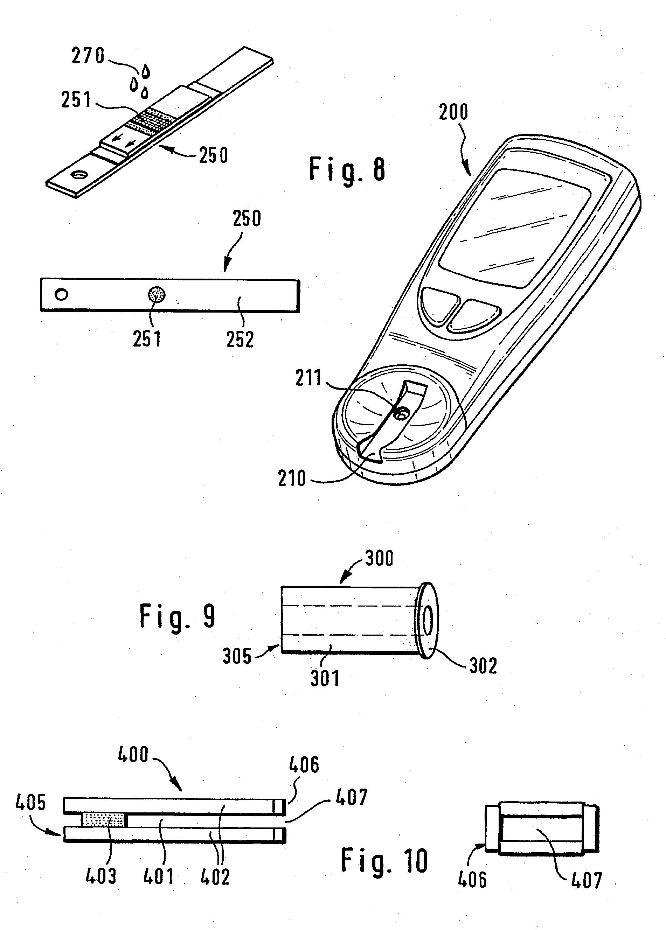

However, the user guidance by illumination of the sample application area can be used in a much wider field as shown in figures 8 to 10.

Figure 8 depicts a reflectometric blood glucose testing device (200) which is sold under the name AccuChek Active. The device has a port (210) for receiving individual test elements (250). A suitable test element is shown from the upper side and the backside. When the test element is arranged for measurement a test element (250) is located in the port (210) so that the backside of the test medium (251) is positioned above an optical sensing unit (211). The test medium changes color in dependence on analyte concentration in a sample fluid (270) which is applied to the upper side of the test medium. The backside of the test medium can be optically accessed through a recess in the test element base (252) to allow optical reading. In prior art systems illumination of the test medium is made for reading only but not for user guidance. Therefore the present invention claims the concept of user guidance for sample application by illuminating an area on the test medium. Advantageously in this context the indication of sample fluid size and guidance for positioning of the sample fluid by use of the illumination site as well as the illuminated area size as described above can be employed. Further it is preferred in this embodiment to employ a continuous illumination of the sample application zone which remains until sample is applied.

In Figure 9 there is shown the illumination for user guidance concept in the context of a light guiding test element (300). The test element has the shape of a tube (301) made of light guiding material (e.g. a clear plastic as polymethylmethacrylate). On the front side of the tube there is located a test medium (301) onto which sample fluid needs to be applied for analysis. The backside of the tube is connected to an optics (not shown) for guiding light (305) into the tube and for receiving light reflected from the backside of the test medium (302). For user guidance illumination can be made in the same way by guiding light into the light guiding test element so that the test medium onto which fluid has to be applied is illuminated. It has to be understood that this invention is not restricted to tube shaped test elements but that it also can be applied to other light guiding test elements as well. Contrary to the conventional use of such light guiding test elements illumination is activated when the testing device is ready for sample reception and the illumination indicates to the user that sample has to be applied.

Figure 10 shows the illumination for user guidance in a further context. The test element (400) is a capillary fill test element having a capillary channel (401) formed in a layer (402) of material. This layer (401) or a layer of material located above or below have light guiding properties. When light (405) is directed into the back end of the test element the frontside (406) of the test element is illuminated. Sample fluid is applied to the capillary end (407) at the frontside and the sample fluid moves through the capillary channel to the test medium (403). The test medium e.g. can be evaluated electrochemically by electrodes contacting the test medium. Alternatively the test medium can be read optically trough a recess in the test element. In this embodiment the sample is not directly applied to the test medium itself but to a transport capillary. The illumination indicates a portion of the test element to which sample has to be applied. However, the sample has to be applied to the capillary which is indicated by the illuminated end of the test element located next to the capillary end.

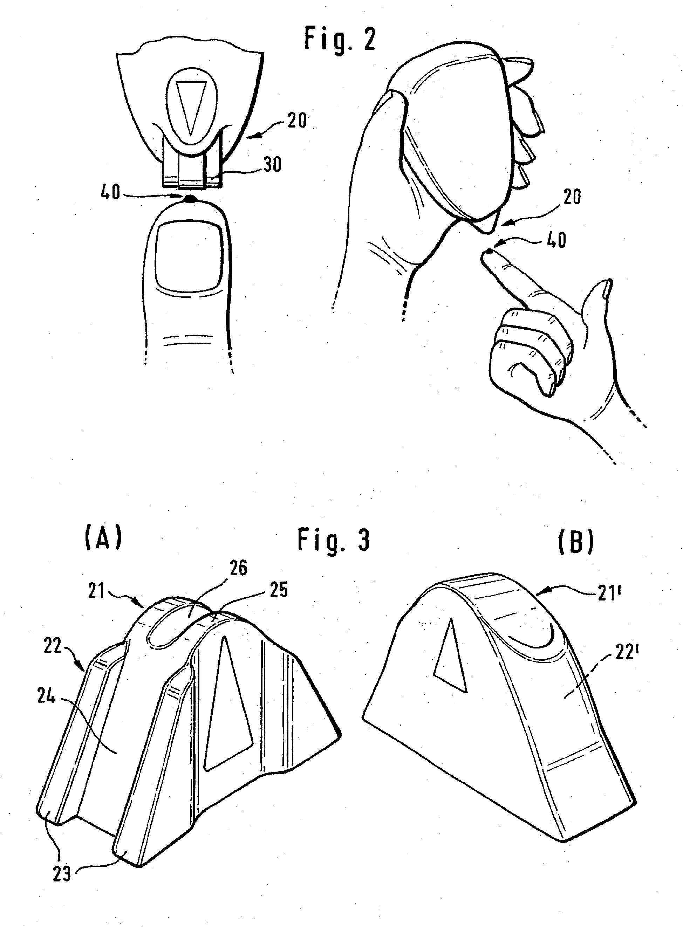

Figure 2 shows the application of body fluid (40) to an exposed portion of test media tape (30) at the convex tip portion (20) of a testing device. From the figures it can be seen how the tip portion facilitates sample application.

Figure 3 depicts two tip portion (20) embodiments in more detail. The embodiment of figure 3A has an exposure portion (21) and a guidance portion (22). The guidance portion (22) has a channel formed by two rails (23) which guide a test media tape for traveling on the bottom surface (24) of the channel. The exposure portion (21) preferably has no protruding elements as e.g. rails which could hinder contacting of the test media tape sited in this area with body fluid. The tape supporting surface (25) further has a recess (26) as depicted in figure 3A. In this recess a sensor can be disposed for evaluation of the test media tape. Suitable sensors are optical sensors including a light source and a detector for measuring light reflected from the test medium. Further a sensor unit with electrodes can be disposed in that recess (26) for contacting electrochemical test media at the exposure portion (21).

It is preferred to locate the sensing portion of a sensor unit at the exposure portion since measurement can be done right after the sample has been applied and no tape transport is necessary before measurement. However, it is also possible to locate a measurement unit for analyte concentration measurement at another position to which the test medium is moved after sample has been applied to it.

In figure 3B a tip portion (20) similar to that of figure 3A is shown. However, the guidance portion (22) has a circumferentially closed channel through which the test media tape runs. The tape is therefore guided by the inner walls of that channel to run over the exposure portion (21'). The exposure portion (21') is different to that of figure 3A in that it has no recess but a sensing unit is integrated into it. This can be accomplished by e.g. making a part or the whole exposure portion (21') from clear plastic to allow light to shine through it and to transmit light reflected from the test medium located over exposure portion (21') to a detector.

The tip portions (20) of figures 3A and 3B can be a part of the testing device (10) or they can be a part of a test media tape cassette.

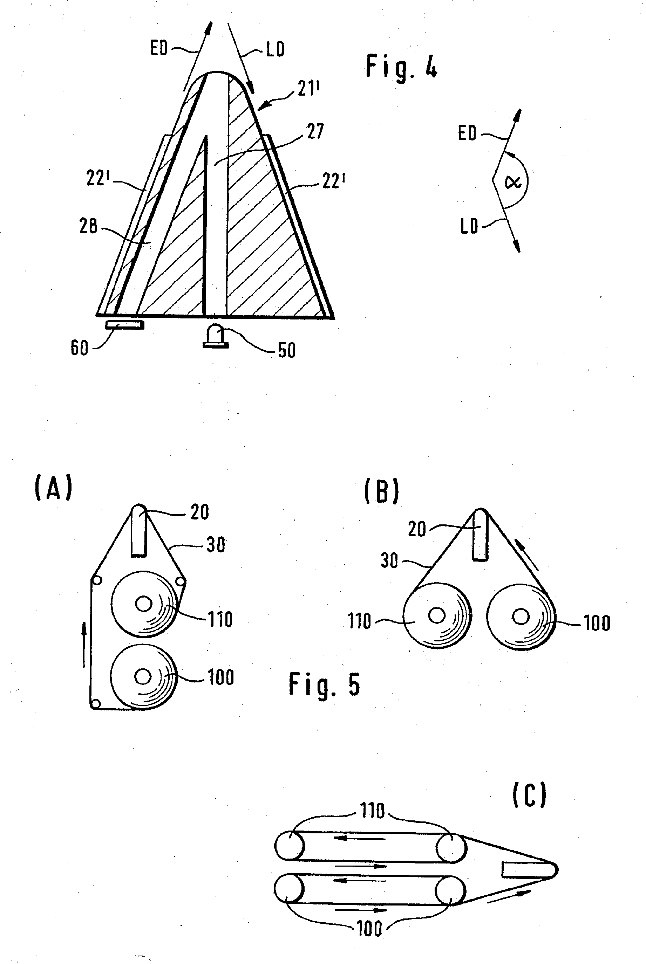

Figure 4 shows a cut through tip portion (20) of figure 3B. In this perspective the guidance portion (22') in form of two channels left and right from the exposure portion (21') can be seen. The tip portion is made from an opaque plastic with an illumination channel (27) and a detection channel (28). At the proximal end of the illumination channel (27) a light source (50) (e.g. a LED) is disposed to illuminate a test medium located above the distal end of the illumination channel. Light reflected from the test medium enters the detection channel (28) at its distal end and is received by a detector (60) (e.g. a photovoltaic element or a photodiode) at the proximal end of the detection channel. Illumination channel and / or detection channel can be empty channels or channels in which optical elements as e.g. lenses or light transmitting fibers are located.

Figure 4 further shows an important effect of the tip portion. The test media tape lies on the exposure portion (21') and enters this portion in the direction shown by a first arrow (ED) and leaves the portion in direction of a second arrow (LD). Right hand to the tip there is shown a vector diagram in which the starting point of these two direction vectors are located on the same spot. It can be seen that there is an angle between leaving direction (LD) and entering direction (ED). It has been found particularly suited to provide handling ease by employing an angle α above 60°, preferably above 90°. It has to be understood that this definition is not restricted to embodiments where the test media tape rests on the exposure portion. This definition is based on the change of direction which is imposed on the test media tape imposed by the tip portion, preferably within the exposure portion.

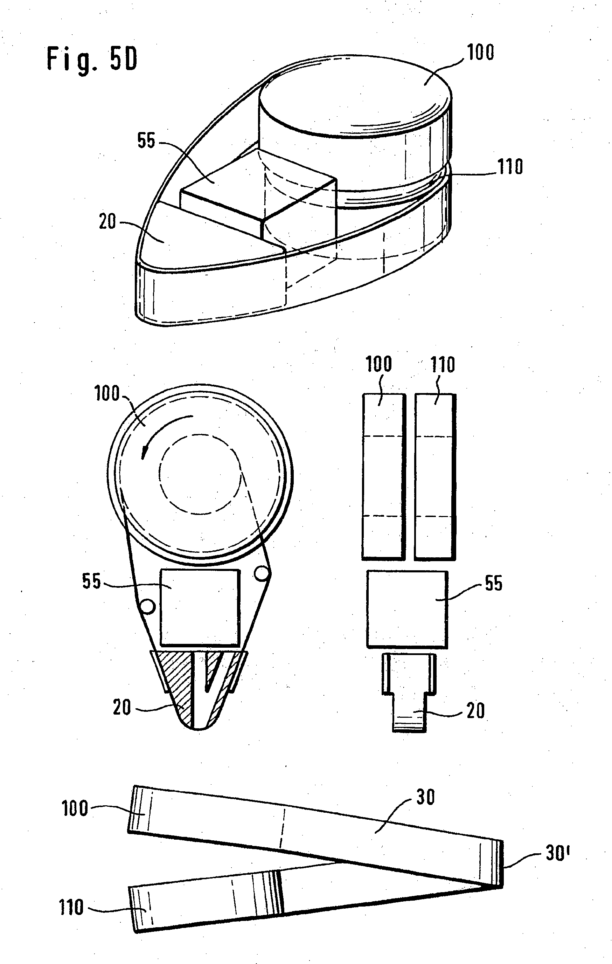

Figures 5A, B, C and D show possible arrangements of supply and storage portions for test media tape relative to the tip portion (20). In figure 5A a test media storage reel (110) is located behind the tip portion (20), and behind the storage reel a supply reel (100) is located. The positions of the supply and the storage reel can be exchanged vice versa while still having this in-line arrangement. The in-line arrangement is advantageous if a slim testing device is desired.

Figure 5B depicts an embodiment where a supply (100) and a storage reel (110) are arranged side by side with the tip portion (20) in between. This arrangement is advantageous if the tip portion (20) is part of the instrument so that the test media tape (30) can be spanned over the tip portion during or after insertion of a test media cassette into the testing device.

Figure 5C depicts a further alternative with two reels in a supply portion and two reels in the storage portion (110). The arrows in all these schematic diagrams indicate the direction of movement of the test media tape during subsequent use of portions of test medium. The embodiment of figure 5C is advantageous if a slim design of the testing device is desired. A view to figure 1 shows that a slim design facilitates user handling.

In figure 5D there is shown an embodiment where the supply and storage reel are arranged coaxially. As can be seen this arrangement is very space efficient. Between the reels and the tip portion (20) there is located an optical unit (55) for evaluating a test medium (30') located at the tip portion.

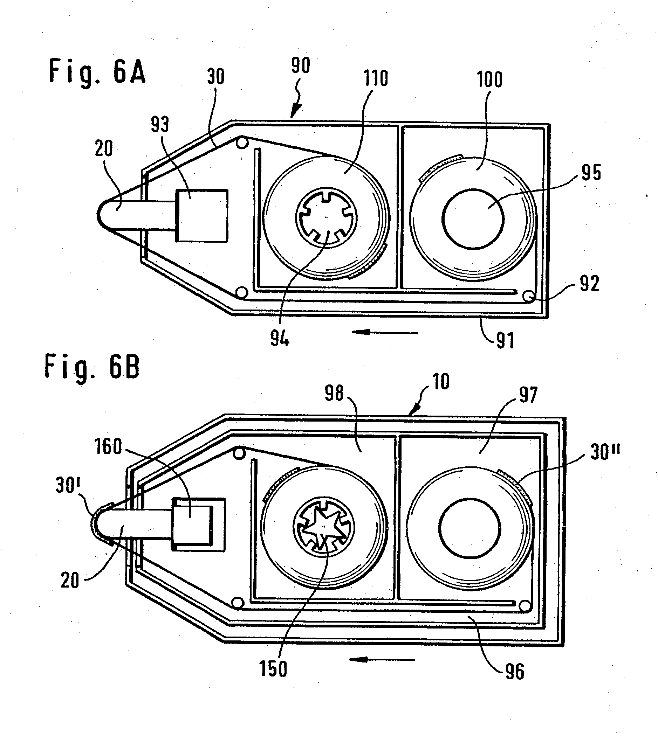

In figure 6A there can be seen a test media cassette (90) for insertion into a testing device. The cassette has a housing (91) in which a supply reel (100) and a storage reel (110) are located. The cassette further comprises a tip portion (20). The test media tape (30) runs over the distal end (outer end) of the tip portion. The test media tape portion on the tip portion is located outside the housing (91) and therefore exposed to the surrounding. At the inner end (distal end) of the tip portion the cassette has a first recess (93) in its housing for receiving an optics belonging to the testing device. It is advantageous that the cassette has rollers or pins (92) for guiding the test media tape through the cassette. In the depicted embodiment the cassette has a second recess (94) for receiving a drive wheel of the testing device. The storage reel (110) has a recess in its center and engagement elements for receiving and engaging said drive wheel. It has to be understood that the drive wheel recess is an option only. Alternative an axis of the storage wheel can be employed which belongs to the cassette and which can be driven from outside the cassette.
The rotational axis (95) of the storage reel belongs to the cassette of the depicted embodiment. However, this axis can also be realized by a recess for receiving an axis belonging to the testing device. The supply reel (100) (or a drive which drives that reel) should have a tension control that controls tension of the test media tape and therefore avoids a too loose or too much tensioned test media tape.

As already mentioned the test media tape is exposed to the environment at the tip portion. Most test media are, however, destroyed or altered by humidity, sunlight etc.. Therefore measures have to be taken to shelter the test media. A first measure is to package the whole cassette (90) before use such that a contact with humidity from the surrounding is prevented. This can be achieved by e.g. a blister package. Bearing in mind that the cassette housing (91) can be made as a body closed against humidity with the exception of the tip region embodiments can be contemplated which employ a humidity proof cover over the tip region which can be removed prior to use of the cassette.

Figure 6B shows a testing device (10) with the cassette (90) of figure 6A being inserted into it. The testing device has a drive wheel (150) engaging the storage reel (110) of the cassette. The drive wheel is driven by a motor (not shown). Alternative the drive wheel can be actuated manually by a user. When a motor is employed this can be actuated by a drive unit which controls and coordinates the transfer of test media into the sampling position on the tip portion (20) and measurement. As already mentioned it is advantageous to conduct an evaluation of a test medium wetted with sample fluid when the test medium is located at the tip portion. However, it is also possible to transfer a test medium wetted with sample fluid away from that position into a spatially distinct evaluation position, e.g. within the cassette. In figure 6B there can be further seen an optics (160) which belongs to the testing device and which enters the first recess (93) when the cassette (90) is inserted into the testing device (10). In case of optical measurement it is necessary to couple the device optics (160) with the tip portion (20). This e.g. can be accomplished by employing a tip portion as an optical transparent tip or to include optical fibers into an otherwise opaque tip (see e.g. figure 4). The device optics (160) can be made by use of fiber optics to which a light source and a detector are coupled.
Figure 6B further shows an important measure to shelter test media against humidity and other influences when the cassette is already in use. As can be seen the distance of test media on the tape is chosen in a way that a second test medium (30") is located inside the cassette housing when a first test medium (30') is located on the tip portion (20). Further it is preferred if the distance between successive test media is so large that a successive test element is still within the sheltering housing when the actual test element is already located within the storage portion of the device. Even more advantageous the distance between two successive test media is so large that the second test medium is covered by overlying tape while the first test medium is on the tip portion. Hence the tape overlying the second test medium is sheltering it.
Figure 6B further shows a channel (96) inside the cassette located between tip portion and supply portion. This channel is the only connection between the storage section (97) and the outside. Through this channel the test media tape runs from the storage reel on its way to the tip portion. Channel (96) serves to limit convections into the channel which would introduce humid air into the storage section in which fresh (unused) test media are stored. Further this channel serves as a diffusion channel which puts a resistance on the diffusion of humidity into the storage section.

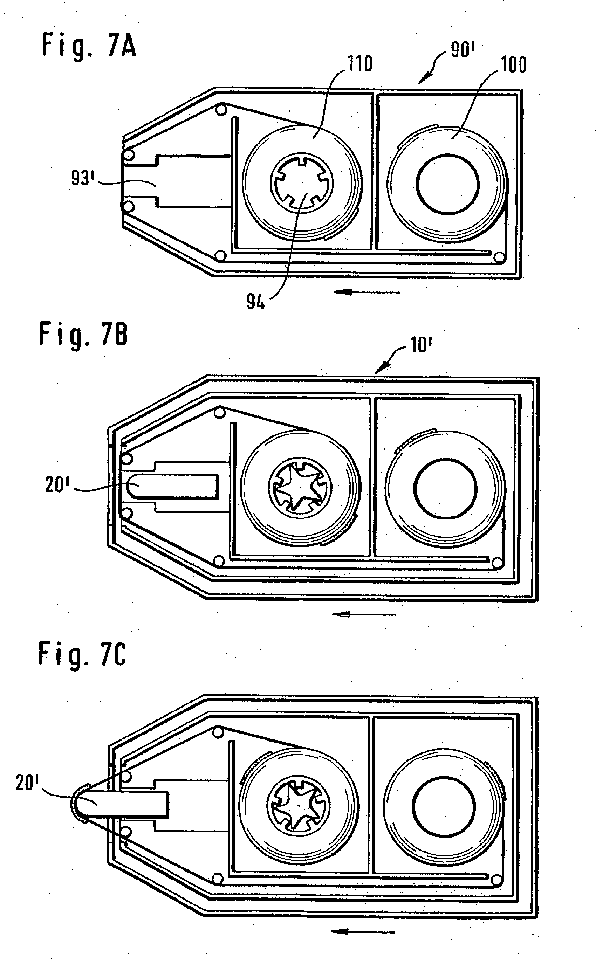

Figure 7 shows an alternative to the embodiment of figure 6. Here the differences between these two concepts will be described and for similarities reference is made to the description of figure 6.
Figure 7A depicts a test media cassette (90') having a supply reel (100) and a storage reel (110). The cassette has a first recess (93') for receiving an optics. This embodiment also has a second recess (94') for receiving a drive wheel similar to that described above. However, contrary to figure 6 the cassette of figure 7 has no tip portion.
Figure 7B shows the cassette of figure 7A inserted into a testing device (10'). As can be seen the testing device has a tip portion which is coupled to an evaluation optics of the testing device. As apparent from figures 7A and 7B the cassette can be inserted into the testing device without user handling steps for guiding the test media tape onto the tip portion. With insertion the exposed portion of test media tape is already located in front of the tip portion (20') belonging to the testing device. From figure 7C it can be seen that the tip portion (20') is moved so that test media tape from the exposed portion is moved outside the device housing. Test media therefore can be accessed very easily by a user at the tip of the tip portion which extends outside the contour of the testing device.

## Claims

1. A body fluid testing device (10) for analyzing a body fluid, comprising:
• a housing
• a test media tape (30) adapted to collect the body fluid,
• a supply portion (100) storing an uncontaminated section of the test media tape,
• a storage portion (110) for storing a contaminated section of the test media tape,
• an exposure portion (21) positioned between the supply portion and the storage portion, the exposure portion being adapted to expose a section of the test media tape to body fluid on a body surface;
said exposure portion has a tip portion (20) for exposing a test medium of said test media tape to body fluid application and said tip portion imposing a change of direction to said tape which is above 60°, and the body fluid testing device further comprising optics coupled with the tip portion,
**characterised in that** said tip portion at least partially is located outside the contour of said housing.

2. The device of claim 1, wherein said tip portion (20) is located at a distal end of the device.

3. The device of claim 1, wherein said test media tape is housed in a cassette (90).

4. The device of claim 1, wherein said tip portion comprises guiding means which guide the test media tape to avoid a slip of the tape off the tip portion.

5. The device of claim 4, wherein said guiding means are one or more guide rails (23) or at least one channel (22').

6. The device of claim 1, wherein said tip portion includes at least one optical channel for illuminating a test medium and / or detecting light reflected by a test medium.

7. The device of claim 1, wherein:
the supply portion includes a supply reel (100), wherein the uncontaminated section of the test media tape (30) is wound on the supply reel; and
the storage portion includes a storage reel (110), wherein the contaminated section of the test media tape can be wound on the storage reel.

8. The device of claim 1, further comprising a piercing device adapted to pierce skin.

9. The device of claim 8, wherein said piercing device is arranged to pierce a body portion located at the tip portion (20).

10. The device of claim 1 further comprising a sensor for sensing a change of a test medium induced by reaction with said body fluid.

11. The device of claim 10, wherein the sensor includes:
a light source and a detector;
a light transmission means coupled to the light source, the transmission means being adapted to transmit light from the light source onto a test medium; and
a light detection means the detection means being adapted to receive reflected light from said test medium and transmits the reflected light to the detector for analysis.

12. The device of claim 1, wherein said tip portion is part of the device.

13. A test cassette (90) for housing test media for sampling body fluid, comprising:
a section for receiving test media tape that is contaminated with past samples of the body fluid and an uncontaminated section;
a housing (91) including a supply portion (97) in which the uncontaminated section of the test media tape (30) is enclosed, the housing further including a storage portion (98) in which a contaminated section of the test media tape can be enclosed, the housing defining an exposure opening at which the test media tape is exposed to the body fluid;
wherein the test cassette further comprises a tip portion (20) which guides the test media tape to expose a portion for body fluid application and said tip portion imposing a change of direction to said tape which is above 60° and the housing having a recess (93) for receiving a sensor (60) belonging to a testing device (10),
**characterised in that** said tip portion at least partially is located outside the contour of the housing.

14. The test cassette of claim 13 further having a humidity proof cover over the tip region.

15. The test cassette of claim 13, wherein said cassette has a channel (96) which forms the sole air connection between the supply portion (97) and the surroundings of the cassette.

16. The cassette of claim 13, wherein successive test media on the test media tape have a distance, said distance being chosen that when a first test medium is located on the tip portion the successive test element is located within the housing (91) of the test media cassette.

17. The cassette of claim 16, wherein said distance is chosen so that when a first test element is located on the tip portion the successive test element is covered by overlying tape.

18. The cassette of claim 15, wherein successive test media on the test media tape have a distance, said distance being chosen that when a first test medium is located on the tip portion the successive test element is located within the storage section (97) being sheltered from humidity by said channel (96).

## Patentansprüche

1. Testgerät (10) für Körperflüssigkeit zur Analyse einer Körperflüssigkeit, umfassend:
- ein Gehäuse
- ein Testmedienband (30) zum Sammeln der Körperflüssigkeit,
- einen Zuführungsabschnitt (100), in dem ein nicht kontaminierter Abschnitt des Testmedienbands aufbewahrt wird,
- einen Aufbewahrungsabschnitt (110) zum Aufbewahren eines kontaminierten Abschnitts des Testmedienbands,
- einen Expositionsabschnitt (21), der zwischen dem Zuführungsabschnitt und dem Aufbewahrungsabschnitt angeordnet ist, wobei der Expositionsabschnitt ausgelegt ist, einen Abschnitt des Testmedienbands Körperflüssigkeit auf einer Körperoberfläche auszusetzen;
wobei der Expositionsabschnitt einen Spitzenabschnitt (20) aufweist, um ein Testmedium des Testmedienbands einer Applikation von Körperflüssigkeit auszusetzen, und wobei der Spitzenabschnitt dem Band eine Richtungsänderung von über 60° auferlegt, und wobei das Testgerät für Körperflüssigkeit ferner eine mit dem Spitzenabschnitt gekoppelte Optik umfasst, **dadurch gekennzeichnet, dass** der Spitzenabschnitt zumindest teilweise außerhalb der Kontur des Gehäuses angeordnet ist.

2. Gerät nach Anspruch 1, worin der Spitzenabschnitt (20) an einem distalen Ende des Geräts angeordnet ist.

3. Gerät nach Anspruch 1, worin das Testmedienband in einer Kassette (90) untergebracht ist.

4. Gerät nach Anspruch 1, worin der Spitzenabschnitt Führungsmittel umfasst, die das Testmedienband führen, um Abrutschen des Bands von dem Spitzenabschnitt zu vermeiden.

5. Gerät nach Anspruch 4, worin das Führungsmittel ein oder mehrere Führungsschienen (23) oder zumindest einen Kanal (22') umfasst.

6. Gerät nach Anspruch 1, worin der Spitzenabschnitt zumindest einen optischen Kanal zur Beleuchtung eines Testmediums und/oder zum Nachweisen von Licht, das von einem Testmedium reflektiert wird, aufweist.

7. Gerät nach Anspruch 1, worin:
der Zuführungsabschnitt eine Zuführungsspule (100) aufweist, worin der nicht kontaminierte Abschnitt des Testmedienbands (30) auf die Zuführungsspule gewickelt ist; und
der Aufbewahrungsabschnitt eine Aufbewahrungsspule (110) aufweist, worin der kontaminierte Abschnitt des Testmedienbands auf die Aufbewahrungsspule gewickelt werden kann.

8. Gerät nach Anspruch 1, ferner eine Einstechvorrichtung umfassend, die zum Einstechen in Haut ausgelegt ist.

9. Gerät nach Anspruch 8, worin die Einstechvorrichtung angeordnet ist, in einen an dem Spitzenabschnitt (20) angeordneten Körperabschnitt einzustechen.

10. Gerät nach Anspruch 1, ferner umfassend einen Sensor zum Erfassen einer Veränderung eines Testmediums, die durch Reaktion mit der Körperflüssigkeit ausgelöst wurde.

11. Gerät nach Anspruch 10, worin der Sensor Folgendes aufweist:
eine Lichtquelle und einen Detektor;
ein mit der Lichtquelle verbundenes Lichtübertragungsmittel, wobei das Übertragungsmittel ausgelegt ist, Licht von der Lichtquelle auf ein Testmedium zu übertragen; und
ein Lichtdetektionsmittel, wobei das Detektionsmittel ausgelegt ist, reflektiertes Licht von dem Testmedium zu empfangen und das reflektierte Licht zu dem Detektor zur Analyse zu übertragen.

12. Gerät nach Anspruch 1, worin der Spitzenabschnitt Teil des Geräts ist.

13. Testkassette (90) zur Unterbringung von Testmedien zur Aufnahme von Körperflüssigkeit, umfassend:
einen Abschnitt zur Aufnahme von Testmedienband, das mit vergangenen Proben der Körperflüssigkeit kontaminiert ist, und einen nicht kontaminierten Abschnitt;
ein Gehäuse (91) mit einem Zuführungsabschnitt (97), in dem der nicht kontaminierte Abschnitt des Testmedienbands (30) eingeschlossen ist, wobei das Gehäuse ferner einen Aufbewahrungsabschnitt (98) aufweist, in dem ein kontaminierter Abschnitt des Testmedienbands eingeschlossen werden kann, wobei das Gehäuse eine Expositionsöffnung definiert, an der das Testmedienband der Körperflüssigkeit ausgesetzt wird;
worin die Testkassette ferner einen Spitzenabschnitt (20) umfasst, der das Testmedienband führt, um einen Teil zur Applikation von Körperflüssigkeit freizulegen, und der Spitzenabschnitt dem Band eine Richtungsänderung von über 60° auferlegt und das Gehäuse eine Aussparung (93) zur Aufnahme eines Sensors (60), der zu dem Testgerät (10) gehört, aufweist, **dadurch gekennzeichnet, dass** der Spitzenabschnitt zumindest teilweise außerhalb der Kontur des Gehäuses angeordnet ist.

14. Testkassette nach Anspruch 13, ferner eine feuchtigkeitsbeständige Abdeckung über der Spitzenregion aufweisend.

15. Testkassette nach Anspruch 13, worin die Kassette einen Kanal (96) aufweist, der die einzige Luftverbindung zwischen dem Zuführungsabschnitt (97) und der Umgebung der Kassette bildet.

16. Testkassette nach Anspruch 13, worin aufeinanderfolgende Testmedien auf dem Testmedienband einen Abstand aufweisen, wobei der Abstand so gewählt ist, dass, wenn sich ein erstes Testmedium auf dem Spitzenabschnitt befindet, das nachfolgende Testelement in dem Gehäuse (91) der Testmedienkassette angeordnet ist.

17. Testkassette nach Anspruch 16, worin der Abstand so gewählt ist, dass, wenn sich ein erstes Testelement auf dem Spitzenabschnitt befindet, das nachfolgende Testelement durch darüber liegendes Band abgedeckt wird.

18. Testkassette nach Anspruch 15, worin aufeinanderfolgende Testmedien auf dem Testmedienband einen Abstand aufweisen, wobei der Abstand so gewählt ist, dass, wenn sich ein erstes Testmedium auf dem Spitzenabschnitt befindet, das nachfolgende Testelement in dem Aufbewahrungsabschnitt (97), der von dem Kanal (96) vor Feuchtigkeit geschützt ist, angeordnet ist.

## Revendications

1. Dispositif de test de liquide corporel (10) pour l'analyse d'un liquide corporel, comprenant :
• un boîtier
• une bande de milieux de test (30) conçue pour recueillir le liquide corporel,
• une partie alimentation (100) stockant une section non contaminée de la bande de milieux de test,
• une partie stockage (110) pour stocker une section contaminée de la bande de milieux de test,
• une partie exposition (21) positionnée entre la partie alimentation et la partie stockage, la partie exposition étant conçue pour exposer une section de la bande de milieux de test au liquide corporel sur une surface du corps ;
ladite partie exposition présente une partie embout (20) pour exposer un milieu de test de ladite bande de milieux de test à l'application d'un liquide corporel et ladite partie embout imposant un changement de direction à ladite bande qui est supérieur à 60 °, et le dispositif de test de liquide corporel comprenant en outre une optique couplée à la partie embout **caractérisée en ce que** ladite partie embout est située au moins en partie à l'extérieur du contour dudit boîtier.

2. Dispositif selon la revendication 1, dans lequel ladite partie embout (20) est située à une extrémité distale du dispositif.

3. Dispositif selon la revendication 1, dans lequel ladite bande de milieux de test est logée dans une cassette (90).

4. Dispositif selon la revendication 1, dans lequel ladite partie embout comporte des moyens de guidage qui guident la bande de milieux de test afin d'éviter un glissement de la bande hors de la partie embout.

5. Dispositif selon la revendication 4, dans lequel lesdits moyens de guidage sont un ou plusieurs rails de guidage (23) ou au moins un canal (22').

6. Dispositif selon la revendication 1, dans lequel ladite partie embout comporte au moins un canal optique pour éclairer un milieu de test et/ou détecter la lumière réfléchie par un milieu de test.

7. Dispositif selon la revendication 1, dans lequel :
la partie alimentation comprend une bobine d'alimentation (100), dans laquelle la section non contaminée de la bande de milieux de test (30) est enroulée sur la bobine d'alimentation ; et
la partie stockage comprend une bobine de stockage (110), dans laquelle la section contaminée de la bande de milieux de test peut être enroulée sur la bobine de stockage.

8. Dispositif selon la revendication 1, comprenant en outre un dispositif de perçage conçu pour percer la peau.

9. Dispositif selon la revendication 8, dans lequel ledit dispositif de perçage est agencé pour percer une partie du corps située au niveau de la partie embout (20).

10. Dispositif selon la revendication 1, comprenant en outre un capteur pour détecter un changement d'un milieu de test induit par la réaction avec ledit liquide corporel.

11. Dispositif selon la revendication 10, dans lequel le capteur comprend :
une source de lumière et un détecteur ;
un moyen de transmission de lumière couplé à la source de lumière, le moyen de transmission étant conçu pour transmettre la lumière provenant de la source de lumière sur un milieu de test ; et
un moyen de détection de lumière, le moyen de détection étant conçu pour recevoir la lumière réfléchie à partir dudit milieu de test et transmettre la lumière réfléchie au détecteur pour analyse.

12. Dispositif selon la revendication 1, dans lequel ladite partie embout fait partie du dispositif.

13. Cassette de test (90) pour loger des milieux de test pour l'échantillonnage de liquide corporel, comprenant :
une section pour recevoir la bande de milieux de test qui est contaminée par d'anciens échantillons du liquide corporel et une section non contaminée ;
un boîtier (91) comprenant une partie alimentation (97) dans laquelle la section non contaminée de la bande de milieux de test (30) est enfermée, le boîtier comprenant en outre une partie stockage (98) dans laquelle une section contaminée de la bande de milieux de test peut être enfermée, le boîtier définissant une ouverture d'exposition au niveau de laquelle la bande de milieux de test est exposée au liquide corporel ;
dans laquelle la cassette de test comprend en outre une partie embout (20) qui guide la bande de milieux de test pour exposer une partie pour l'application de liquide corporel et ladite partie embout imposant un changement de direction à ladite bande qui est supérieur à 60 °, et le boîtier présentant un renfoncement (93) destiné à recevoir un capteur (60) appartenant à un dispositif de test (10), **caractérisée en ce que** ladite partie embout est située au moins en partie à l'extérieur du contour du boîtier.

14. Cassette de test selon la revendication 13, présentant en outre un couvercle étanche à l'humidité sur la région d'embout.

15. Cassette de test selon la revendication 13, dans laquelle ladite cassette comporte un canal (96) qui forme l'unique connexion à l'air entre la section alimentation(97) et l'environnement de la cassette.

16. Cassette selon la revendication 13, dans laquelle les milieux de test successifs sur la bande de milieux de test ont une distance, ladite distance étant choisie de sorte que, lorsqu'un premier milieu de test est situé sur la partie embout, l'élément de test suivant est situé dans le boîtier (91) de la cassette de milieux de test.

17. Cassette selon la revendication 16, dans laquelle ladite distance est choisie de sorte que, lorsqu'un premier élément de test est situé sur la partie embout, l'élément de test suivant est recouvert par une bande sus-jacente.

18. Cassette selon la revendication 15, dans laquelle les milieux de test successifs sur la bande de milieux de test ont une distance, ladite distance étant choisie de sorte que, lorsqu'un premier milieu de test est situé sur la partie embout, l'élément de test suivant est situé dans la section stockage (97) étant protégé de l'humidité par ledit canal (96).
